# EUROPEAN PATENT SPECIFICATION

(11) **EP 1 121 151 B1**
(45) Date of publication and mention of the grant of the patent: **13.12.2006**
(21) Application number: 99956441.2
(22) Date of filing: 07.10.1999
(51) Int. Cl.: A61K 39/395, C07K 16/28, C07K 14/705, A61P 37/06

(54) **ANTI-INFLAMMATORY ALPHA2, BETA1 INTEGRIN-RELATED SUBSTANCES OR ANTIBODIES**
ANTI-ENTZÜNDLICHE ALPHA2, BETA1 INTEGRIN-VERWANDTE SUBSTANZEN ODER ANTIKÖRPER
SUBSTANCES OU ANTICORPS ANTI-INFLAMMATOIRES APPARENTEES A L'ALPHA 2 BETA 1 INTEGRINE

(30) Priority: 07.10.1998 SE 9803428; 07.10.1998 US 103558 P
(43) Date of publication of application: 08.08.2001
(73) Proprietor: Lindbom, Lennart, 756 45 Uppsala (SE); Werr, Joachim, 113 26 Stockholm (SE)
(72) Inventor: LINDBOM, Lennart, S-756 45 Uppsala (SE); WERR, Joachim, S-113 37 Stockholm (SE)
(74) Representative: Larfeldt, Helene
(86) International application number: PCT/SE1999/001801
(87) International publication number: WO 2000/020030

(56) References cited:
- EP-A1- 0 759 302
- EP-A1- 0 879 602
- US-A- 5 516 634
- US-A- 5 538 724
- DATABASE MEDLINE [Online] HARLER M.B. ET AL.: 'Promotion of neutrophil chemotaxis through differential regulation of beta 1 and beta 2 integrins', XP002945108 Retrieved from Dialog Information Service, File 155, accession no. 09980407 Database accession no. 99282968 & J. IMMUNOL. vol. 162, no. 44, 01 June 1999, (UNITED STATES), pages 6792 - 6799
- JOACHIM WERR ET AL.: 'Beta1 Integrins Are Critically Involved in Neutrophil Locomotion in Extravascular Tissue In Vivo' J. EXP. MED. vol. 187, no. 12, June 1998, pages 2091 - 2096, XP001013205
- DATABASE MEDLINE [Online] PISCHEL K.D. ET AL.: 'Platelet glycoproteins Ia, Ic and IIa are physicochemically indistinguishable from the very late activation antigens adhesion-related proteins of lymphocytes and other cell types', XP002945109 Retrieved from Dialog Information Service, File 155, accession no. 05494878 Database accession no. 88116005 & J. CLIN. INVEST. vol. 81, no. 2, February 1988, (UNITED STATES), pages 505 - 513
- DATABASE MEDLINE [Online] GOLDMAN R. ET AL.: 'VLA-2 is the integrin used as a collagen receptor by leukocytes', XP002945110 Retrieved from Dialog Information Service, File 155, accession no. 07276020 Database accession no. 92249399 & EUR. J. IMMUNOL. vol. 22, no. 5, May 1992, (GERMANY), pages 1109 - 1114
- DATABASE MEDLINE [Online] KNIGHT C.G. ET AL.: 'Identification in collagen type I of an integrin alpha2 beta1-binding site containing an essential GER sequence', XP002945111 Retrieved from Dialog Information Service, File 155, accession no. 09791528 Database accession no. 99057885 & J. BIOL. CHEM. vol. 273, no. 50, 11 December 1998, (UNITED STATES), pages 33287 - 33294
- DATABASE MEDLINE [Online] SAELMAN E.U. ET AL.: 'Platelet adhesion to collagen type I through VIII conditions of stasis and flow is mediated by GPIa/IIa (alpha 2 beta 1-integrin)', XP002945112 Retrieved from Dialog Information Service, File 155, accession no. 07871565 Database accession no. 94162622 & BLOOD vol. 83, no. 5, 01 March 1994, (UNITED STATES), pages 1244 - 1250
- DATABASE MEDLINE [Online] LUGUE A. ET AL.: 'Activated conformations of very late activation integrins directed by a group of antibodies (HUTS) specific for a novel regulatory region (355-425) of the common beta 1 chain', XP002945113 Retrieved from Dialog Information Service, File 155, accession no. 08684577 Database accession no. 96212165

## Description

### Technical field

The present invention relates to the field of anti-inflammatory medicaments and more specifically to use of integrin blocking compounds in the manufacture of such medicaments.

### Background

An inflammatory response is a response to infection or tissue damage and is designed to localize invading microorganisms and arrest the spread of infection. During inflammation, blood vessels in the area of inflammation are dilated thus increasing blood circulation, allowing increased number of phagocytotic blood cells to reach the affected area. Further, inflammatory responses are also involved in autoimmune conditions, in which case they are however undesired.

In the initial phase of inflammation, phagocytic white blood cells, such as polymorphonuclear leukocytes (PMN), are most abundant, while monocytes and macrophages predominate in the later stages. More specifically, the recruitment of PMN constitutes the first line of defense in the cellular inflammatory response (Springer TA: Traffic signals for lymphocyte recirculation and leukocyte emigration: the multistep paradigm. Cell 76:301, 1994). Following their emigration from the vasculature, these cells respond to chemotactic gradients by moving in the extravascular tissue toward sites of injury or infection. The locomotion of extravasated PMN is thought to depend on coordinated and transient interactions of leukocytic cell adhesion molecules with extracellular matrix (ECM) components (Downey GP: Mechanisms of leukocyte motility and chemotaxis. Curr. Opin. Immunol. 6:113, 1994). While it is well established that adhesion molecules of the selectin and β₂ integrin families are critical for PMN adhesion to the endothelium (Carlos TM, Harlan JM: Leukocyte-endothelial adhesion molecules. Blood 84:2068, 1994), there are no direct evidences that PMN migration in the extravascular tissue is dependent on these molecules.

β₁(VLA) integrins comprise a family of receptors that mediate cell adhesion to ECM proteins (e.g. collagen, fibronectin and laminin). They share a common β-chain (β₁; CD29) that is non-covalently linked to one of at least six different α-chains (α₁-α₆; CD49a-f) determining the binding properties of the receptor (Hemler ME: VLA proteins in the integrin family: structures, functions, and their role on leukocytes. Annu. Rev. Immunol. 8:365, 1990). β₁ integrin function in PMN has been considered to be limited because circulating blood PMN, in contrast to other hematopoietic cells, express only low levels of β₁ integrins (Hemler ME: VLA proteins in the integrin family: structures, functions, and their role on leukocytes. Annu. Rev. Immunol. 8:365, 1990). However, recent observations indicate that extravasation of PMN may be associated with upregulation of β₁ integrins on the cell surface (Kubes P, Niu XF, Smith CW, Kehrli ME Jr, Reinhardt PH, Woodman RC: A novel beta 1-dependent adhesion pathway on neutrophils: a mechanism invoked by dihydrocytochalasin B or endothelial transmigration. FASEB J. 9:1103, 1995; Reinhardt PH, Ward CA, Giles WR, Kubes P: Emigrated rat neutrophils adhere to cardiac myocytes via α₄ integrin. Circ. Res. 81:196, 1997; Werr J, Xie X, Hedqvist P, Ruoslahti E, Lindbom L: β₁ integrins are critically involved in neutrophil locomotion in extravascular tissue in vivo. J. Exp. Med. 187:2091, 1998), and significant expression of α₄β₁, α₅β₁ and α₆β₁ on these cells has been reported (Kubes et al., *supra;* Reinhardt et al., *supra;* Werr et al., *supra;* Bohnsack JF, Zhou XN: Divalent cation substitution reveals CD18- and very late antigen-dependent pathways that mediate human neutrophil adherence to fibronectin. J. Immunol. 149:1340, 1992). A critical role for β₁ integrins in PMN locomotion *in vivo* has previously been documented (Werr et al., *supra).* Moreover, this process was shown to involve members of the β₁ integrin family other than the fibronectin-binding receptors α₄β₁ and α₅β₁.

EP 0 759 302 describes a remedy for rheumatoid arthritis wherein an antibody recognising the extracellular region of human VLA-2 is used. Preferablya monoclonal antibody recognizing specifically an extracellular region of the α-chain of human VLA-2. This document does not describe any target cells or mechanisms for the therapeutic effect of VLA-2 blockage in rheumatoid arthritis. As VLA-2 is widely present on both tissue cells (i.e. in the joint) and leukocytes no conclusions can be drawn about the mechanism behind the claimed effect of VLA-2 blockage based on the information given in this document.

US 5,53 8,724 describes compositions and methods for modulating the ability of leukocytes to extravasate through endothelial walls by affecting the ability of the leukocytes to bind to surface membrane proteins (addressins) and endothelial cells by controlling binding between homing receptors and addressins. This means that leukocyte extravasation is blocked intravascularly. Blockage of leukocyte extravasation extracellularly is not discussed in this document.

At present, there are no therapies available based on the modulation of leukocyte motility for the treatment of inflammatory conditions. In order to design such therapies, a further understanding of the inflammatory process, and specifically of the extravasation of leukocytes, is needed. Up to now, no specific receptor utilized by polymorphonuclear leukocytes (PMN) for locomotion in extravascular tissue has been identified.

### Summary of the invention

According to the present invention, expression of α₂β₁ integrin on extravasated PMN and a novel role of this receptor in regulating the extravascular phase of leukocyte trafficking in inflammation have been identified for the first time. Thus, the present invention relates to various advantageous pharmaceutical applications of the present receptor and and modulation of leukocyte motility and/or recruitment, e.g. by ligands binding thereto, as defined in the appended claims.

### Brief description of the drawings

Fig 1 illustrates FACS^{®} analysis of integrin molecule expression on human *(a)* and rat (*b*) PMN.
Fig 2 shows an enhanced transmitted light image of fMLP (10⁻⁷ M) stimulated human PMN adhering to collagen (a), and corresponding image in laser emitted fluorescent light showing immunofluorescent staining for α₂β₁ (b).
Fig 3 illustrates the time course effects of local treatment with antibodies (a) or peptides (b) on PAF-stimulated (10⁻⁷M) PMN locomotion in the rat mesentery.
Fig 4 illustrates fMLP-stimulated (10⁻⁷ M) PMN locomotion in gels of collagen (a) and gelatin (b).
Fig 5 shows the effect of anti-α₂ mAb (Ha1/29) on PMN recruitment in mouse subcutaneous air pouch.
Fig 6 illustrates the effect of an α₂β₁ integrin-binding peptide RKK on chemoattractant induced PMN recruitment in the mouse air pouch.

### Definitions

"α₂β₁ (VLA-2) integrin" is a heterodimeric receptor molecule consisting of an α-chain (CD49b) non-covalently linked to a β-chain (CD29).

The term "α₂β₁-binding compound" refers to a substance that binds preferentially to the α₂β₁ integrin

"RKK" refers to a peptide containing the amino acid sequence arginine-lysine-lysine.

"DGEA" refers to a peptide containing the amino acid sequence aspartate-glycine-glutamine-alanine.

The term "antibody" refers to a polypeptide substantially encoded by an immunoglobulin gene or immunoglobulin genes, or fragments thereof which specifically bind and recognize an analyte (antigen).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e.g.*, an enzyme, toxin, hormone, growth factor, drug, *etc.;* or (b) the variable region, or a portion thereof, is altered, replaced or exchanged with a variable region having a different or altered antigen specificity.

The term "immunoassay" is an assay that utilizes an antibody to specifically bind an analyte. The immunoassay is characterised by the use of specific binding properties of a particular antibody to isolate, target, and/or quantify the analyte.

### Detailed description of the invention

Thus, the present inventors have for the first time shown that surface expression of the α₂β₁ (VLA-2) integrin is induced in human and rat PMN upon extravasation *in vivo,* and that PMN locomotion and recruitment to extravascular tissue is critically dependent on α₂β₁ integrin function, as disclosed in further detail in the section "Experimental" below.

Accordingly, in a first aspect, the present invention relates to the manufacture of a medicament as defined in claims 1-8 aimed at a modulation of leukocyte motility, such as recruitment, in a subject. In this context, it is to be understood that the present medicament is effective to reduce, and preferably eliminate, cell recruitment to an area affected by inflammation, which is achieved by a reduction of the leukocyte motility. The subject leukocyte may be any granular or nongranular white blood cell that presents α₂β₁ integrin, such as a monocyte or a lymphocyte. In one particular embodiment, the leukocyte is a polymorphonuclear leukocyte (PMN). Even though the presence of α₂β₁ integrin has indeed been shown on some cells, the function thereof in the trafficking of leukocytes has never been suggested in the prior art. Accordingly, the effect of the medicament prepared according to the invention is novel and surprising. More specifically, the α₂β₁ integrin suppressor or inhibitor, which is capable of providing an essential or total blocking of the motility function, is included in the medicament. The present blocking of the function is achieved directly, such as by function-inhibition by binding to, α₂β₁ integrin.

As regards substances having a direct effect, they are effective by binding to α₂β₁ integrin, and they too may be any kind of molecule or compound that is capable of such binding, such as the above mentioned, and may also be obtained by screening a suitable library. Screening methods useful in the present context are well known to the skilled in this field. In one embodiment, said suppressor or inhibitor is an antibody, such as a polyclonal, monoclonal, chimeric or anti-idiotypic antibody, or a functional fragment thereof. In an alternative embodiment, said suppressor or inhibitor is an α₂β₁ integrin-binding compound, such as a peptide. Examples of peptides effective to this end is a peptide comprising the amino acid sequence RKK, which is disclosed in detail in Ivaska et al., J. Biol. Chem. 274:3513-3521, 1999, or the amino acid sequence DGEA. In the most preferred embodiment, said antibody is a monoclonal antibody. Thus, in a specific embodiment, the medicament produced is capable of providing a suppression, blocking or elimination of the motility and thereby the recruitment of leukocytes when administered to a patient in need of therapy. Said therapy may more specifically be aimed at the treatment and/or prevention of an inflammatory disease, such as asthma, psoriasis, or IBD (ulcerative colitis, Crohns disease).

Methods of producing antibodies are known to persons of skill in this field. However, a general overview of the techniques available will be given below. To produce antibodies specifically reactive with α₂β₁ integrin, a number of immunogens are used. Epitopes of 8-15, preferably 10, amino acids in length, or greater are the preferred polypeptide immunogen (antigen) for the production of monoclonal or polyclonal antibodies. Thus, the present α₂β₁ integrin, or a synthetic version or an epitope thereof, is injected into an animal capable of producing antibodies. Either monoclonal or polyclonal antibodies can then be generated for subsequent use in pharmaceutical preparation, as libraries in screening methods or in immunoassays to measure the presence and/or quantity of the polypetide.

Specific monoclonal and polyclonal antibodies will usually bind with a K_{D} of at least about 0.1 mM, more usually at least about 50 µM, and most preferably at least about 1 µM or better.

For methods of producing polyclonal antibodies, see *e.g*., Coligan (1991) *Current Protocols in Immunology* Wiley/Greene, NY; and Harlow and Lane (1989) *Antibodies: A Laboratory Manual* Cold Spring Harbor Press, NY).

Monoclonal antibodies are prepared from cells secreting the desired antibody. These antibodies are screened for binding α₂β₁ integrin, or screened for agonistic or antagonistic activity. Techniques for preparing monoclonal antibodies are e.g. found in Stites *et al.* (eds) *Basic and Clinical Immunology* (4th ed.) Lange Medical Publications, Los Altos, CA, and references cited therin; Harlow and Lane, *supra;* Goding (1986) *Monoclonal Antiboides: Principles and Practice* (2d ed.) Academic Press, New York, NY; and Kohler and Milstein (1975) *Nature* 256: 495-497.

The production of suitable α₂β₁ integrin-binding peptides, such as RKK or DGEA, may be performed by the skilled in this field and includes synthetic and recombinant-DNA-methods. Synthetic methods include e.g. solid phase techniques, see e.g. Stewart et al., Solid Phase Peptide Synthesis, 2^{nd} ed., Pierce Chem. Co., Rockford, III. (1984). For a general overview of recombinant-DNA-techniques, see e.g. Sambrook et al., Molecular Cloning A Laboratory Manual, 2^{nd} ed., Vol. 1-3, Cold Spring Harbor Laboratory, Cold Spring Harbor, NY, 1989.

In a second aspect, the present invention relates to a pharmaceutical preparation as defined in claims 9-11. Thus, all aspects discussed above as regards the nature of the effective components of a medicament or pharmaceutical preparation are also relevant here as well as below in the context of methods of treatment. Accordingly, the present preparation comprises a suppressor or inhibitor of the function of α₂β₁ integrin, which is a α₂β₁ integrin-binding peptide, such as one containing the RKK or DGEA sequence, and a pharmaceutically acceptable carrier. In a preferred embodiment, especially for conditions such as IBD, psoriasis and asthma, the present pharmaceutical preparation is administered locally, e.g. cutaneously, intradermally, rectally, or by inhalation. Local treatment may be particularly advantageous in order to avoid undesired secondary effects. Alternatively, it may be in a solution form suitable for injection. A brief review of methods of drug delivery is given in e.g. Langer, Science 249:1527-1533 (1990). The administration is preferably local, i.e. the preparation is given in a way that enables the active components thereof to reach the affected area without being substantially inactivated.

The amount of active ingredient combined with a carrier to produce a single dosage form will vary depending on the subject treated, e.g. body weight, age etc., the condition in question, the particular mode of administration and other factors. The present carrier may be any carrier that is suitable for use together with a specific active component for the treatment of a specific condition and is easily chosen by the one skilled in this field. The carrier may be aqueous, a fat emulsion or of any other suitable nature. In addition to the carrier, the preparation may optionally also comprise an inert diluent, one or more adjuvants, such as wetting agents, substances to approximate physiological pH conditions, toxicity adjusting agents etc. The preparation is sterile and free of any undesirable matter. Pharmaceutical formulations are found e.g. in Remington's Pharmaceutical Sciences, Mack Publishing Company, Philadelphia, PA, 17^{th} ed (1985). An alternative embodiment of the present preparation is a freeze- or spray-dried form, which is to be reconstituted immediately prior to use.

In a method of preparing a pharmaceutical preparation as described above,
(a) a library of suitable chemical substances is screened for α₂β₁ integrin blocking compounds;
(b) one compound is selected and isolated;
(c) said compound is associated with a pharmaceutically acceptable carrier and optionally one or more excipients.

More specifically, said library or pool is comprised of peptides, small organic molecules or substances of any other suitable nature. As mentioned above, the carrier may be a liquid or a finely divided solid. Further, the product may as a final step be shaped into the desired form.

One especially advantageous aspect of the present invention is the use of α₂β₁ integrin as a research tool and an α₂β₁ integrin function blocking and/or inhibiting compound identified as disclosed above. Said compound may be an antibody or α₂β₁ integrin-binding peptide, such as RKK or DGEA, that binds to α₂β₁ integrin with a sufficient specificity to essentially block and suppress the function thereof, preferably by a total inhibition.

In a further aspect, the present invention also relates to a method of *in vitro* diagnosis, wherein
(a) an α₂β₁ integrin suppressing or inhibiting compound is contacted with a suitable sample;
(b) the amount of α₂β₁ integrin bound by said compound is measured,
said amount being an indication on the progression and stage of an inflammatory condition.

In the present application, a "subject in need of anti-inflammatory therapy" may be a human, a human organ or any other susceptible mammal or mammalian organ.

### Detailed description of the drawings

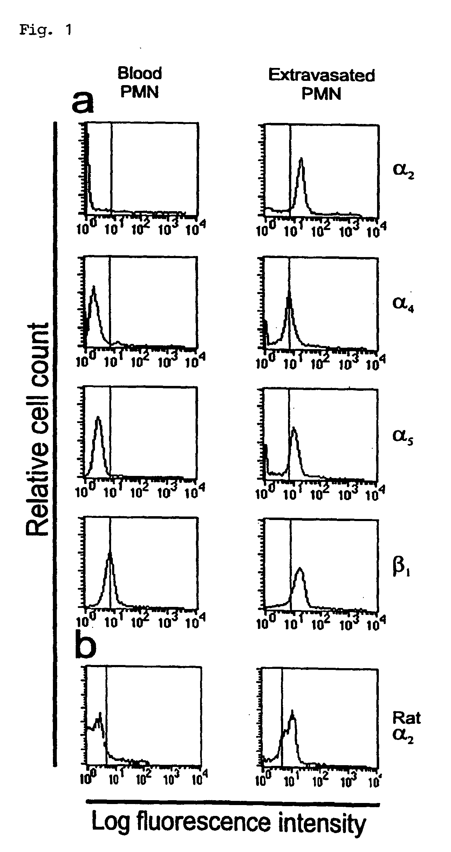

Fig 1 shows a FACS^{®} analysis of integrin molecule expression on human (a) and rat (b) PMN. Left panel shows staining of PMN isolated from peripheral whole blood. Right panel shows staining of extravasated human PMN accumulated in skin blister chambers in response to stimulation with autologous serum and rat PMN accumulated in the peritoneal cavity in response to PAF stimulation (10⁻⁷M). Vertical line indicates the 99th percentile of fluorescence events for cells stained with irrelevant, species-matched IgG. (Histograms are representative tracings of 4-6 analyses for each antibody.)

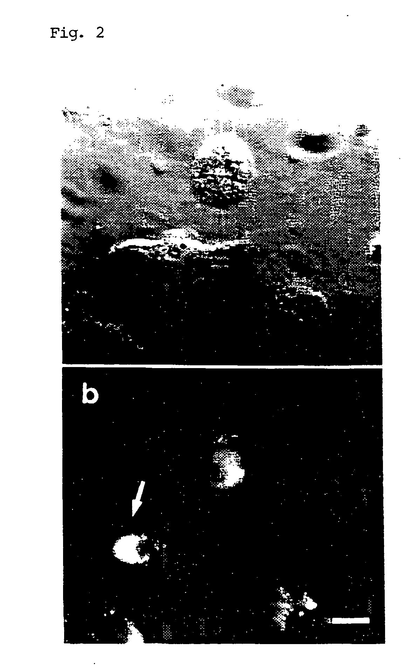

Fig 2 is an enhanced transmitted light image of fMLP (10⁻⁷ M) stimulated human PMN adhering to collagen (a), and corresponding image in laser emitted fluorescent light showing immunofluorescent staining for α₂β₁ (b). Combined examination of cell morphology and α₂β₁ integrin expression revealed that the receptor was localized to the leading edge of most PMN with a distinct head-tail morphology (arrow). Bar indicates 5 µm.

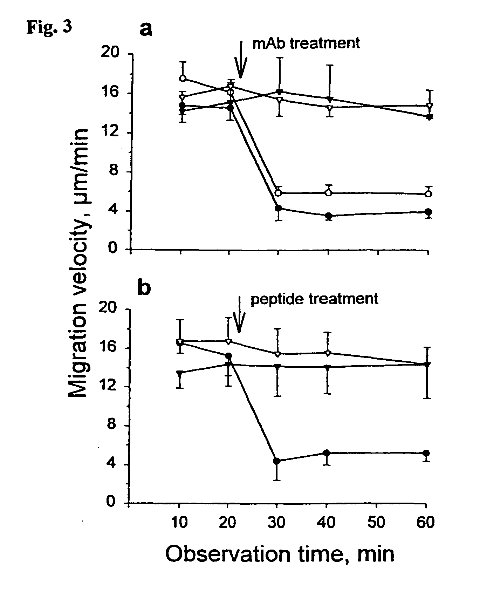

Fig 3 shows the time course effects of local treatment with antibodies (a) or peptides (b) on PAF-stimulated (10⁻⁷M) PMN locomotion in the rat mesentery. a) anti-α₂ (mAb Hal/29) (•); combined anti-α₂ and anti-β₁ (mAb HMβ1-1) (o); anti-α₄ (mAb TA-2) (▼); and anti-α₅ (mAb HMα5-1) (∇) integrin antibodies (100 µg/ml). b) α₂β₁-binding peptide DGEA (25 mM) (•); α₄β₁-binding peptide SLIDIP (▼), and α₅β₁-binding peptide RGDGW (V) (500 µM). Data are based on calculation of mean migration velocity during 10-20 min intervals and presented as mean ± SD of 4-5 experiments for each reagent tested.

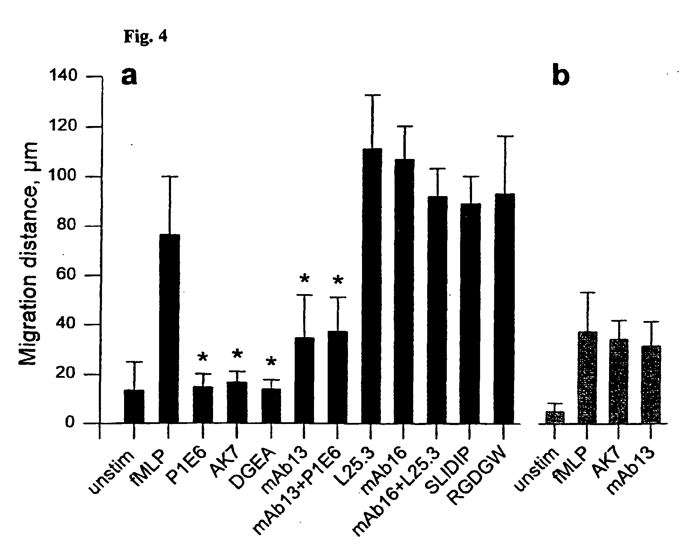

Fig 4 shows fMLP-stimulated (10⁻⁷ M) PMN locomotion in gels of collagen (a) and gelatin (b). Effect of antibodies against α₂ (mAb P1E6 and mAb AK7), α₄ (mAb L25.3), α₅ (mAb16) and β₁ (mAb13) integrin molecules (20 µg/ml), and of integrin-bidning peptides recognizing α₂β₁ (DGEA, 5 mM), α₄β₁ (SLIDIP, 100 µM), and α₅β₁ (RGDGW, 100 µM). Data are based on calculation of migration distance of the leading front during a 30 min incubation period at 37° C and presented as mean ± SD of 5-7 experiments for each combination analyzed. * indicates significant difference vs. fMLP stimulation alone (p< 0.001)

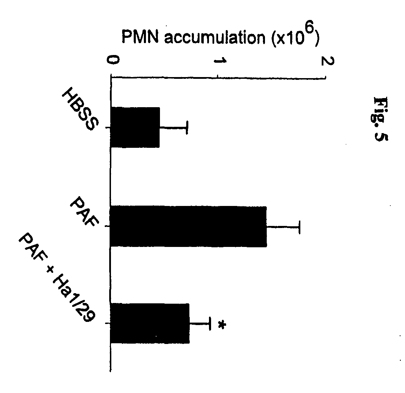

Fig 5 shows the effect of anti-α₂ mAb (Hal/29) on PMN recruitment in mouse subcutaneous air pouch. PMN accumulation was assessed by counting leukocytes in the lavage fluid after 4 h stimulation with HBSS, PAF (10⁻⁷M) together with isotype matched control antibody, (50 µg/ml) or with PAF together with mAb Hal/29 (50 µg/ml). Values are mean ± SD of 7 separate experiments in each group. * indicates significant difference vs. PAF stimulation alone (p< 0.001).

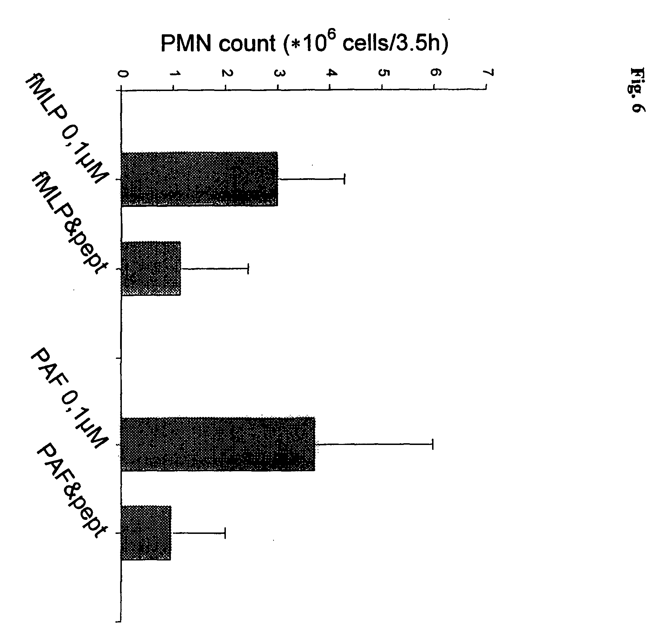

Fig 6 shows the effect of the cyclic peptide CTRKKHDNAQC (RKK) on PMN recruitment in mouse subcutaneous air pouch. PMN accumulation was assessed by counting leukocytes in the lavage fluid after 4 h stimulation with HBSS, PAF (10⁻⁷ M) or with PAF together with RKK (1 mM). Values are mean ± SD of 4 separate experiments in each group.

Further reference to the drawings will be made in the experimental section below, especially in connection with the discussion of the results.

### EXPERIMENTAL

Below, the present invention will be described in detail by way of examples given only as an illustration of the invention and not to be construed as limiting the scope thereof in any way.

### Methods:

*Antibodies and peptides.* The following antibodies reacting with rat integrin molecules were used: Monoclonal antibody (mAb) HMβ1-1 against the β₁ subunit (CD29), and mAb Hal/29 (cross-reactive with mouse) against the α₂ subunit (CD49b) (both from Pharmingen, San Diego, CA), mAb TA-2 (Serotec, Oxford, England) against the α₄ subunit (CD49d) and mAb HMα5-1 (Pharmingen) against the α₅ subunit (CD49e). The following mAb: s reacting with human integrin molecules were used: mAb13 against the β₁ subunit (CD29) (Becton Dickinson, Mountain View, CA), mAb P1E6 (Becton Dickinson) and mAb AK7 (Pharmingen) against the α₂ subunit (CD49b), mAb L25.3 (Becton Dickinson) against the α₄ subunit (CD49d) and mAb 16 against the α₅ subunit (CD49e) (Becton Dickinson). Function-blocking activity has been documented for all antibodies listed above.

The following integrin-binding peptides were used: DGEA (Peninsula Lab., Belmont, CA) specifically blocking α₂β₁ integrin binding to type I collagen (Staatz WD, Fok KF, Zutter MM, Adams SP, Rodriguez BA, Santoro SA: Identification of a tetrapeptide recognition sequence for the alpha 2 beta 1 integrin in collagen. J. Biol. Chem. 266:7363, 1991), SLIDIP blocking the function of α₄β₁ integrin and ACRGDGWMCG (RGDGW) blocking the function of α₅β₁ integrin (Koivunen E, Gay DA, Ruoslahti E: Selection of peptides binding to the alpha 5 beta 1 integrin from phage display library. J. Biol. Chem. 268:20205, 1993).

Antibodies Hal/29, TA-2, HMα5-1, P1E6, AK7, mAb13, L25.3 and mAb16 contained sodium azide (0.001-0.01 % in final dilution). Other reagents were free of preservatives. Control experiments showed that sodium azide at corresponding concentrations was without effect on the parameters analyzed as previously reported (Werr et al., *supra).*

*Isolation of human PMN.* The suction-blister chamber technique was used as previously described (Raud J, Hallden G, Roquet A, van Hage-Hamsten M, Alkner U, Hed J, Zetterström O, Dahlén SE, Hedqvist P, Grönneberg R: Anti-IgE-induced accumulation of leukocytes, mediators, and albumin in skin chamber fluid from healthy and atopic subjects. J. Allergy Clin. Immun. 97:1151, 1996). The experimental procedure was approved by the local ethical committee. In brief, chambers with a volume of 1 ml were mounted on the freshly formed skin blisters on volar aspect of the arm and filled with autologous serum (70 % in HBSS) to stimulate PMN extravasation. The skin blister fluid was collected after 8 h and PMN that had accumulated in the fluid (6-10×10⁶ cells/chamber) were washed and resuspended in HBSS. Peripheral blood PMN were isolated from whole blood by single-step density centrifugation over Polymorphprep (Nycomed Pharma AS, Oslo, Norway). Following hypotonic lysis of contaminating red blood cells, the PMN were washed and resuspended in HBSS.

*Isolation of rat PMN.* PMN extravasation in the peritoneal cavity of Wistar rats (250 g) was induced by i. p. stimulation with platelet-activating factor (PAF; 10⁻⁷ M) (Sigma, St Louis, MO) in 10 ml HBSS. After 2 h, the animals were killed with methyl-ether and peritoneal leukocytes were harvested by washing the peritoneal cavity with 10 ml ice-cold HBSS. The leukocytes were fixed directly in 4 % paraformaldehyde for 5 min at room temperature, washed twice in HBSS, and stained for FACS^{®}-analysis as described. EDTA-anticoagulated blood was collected from the same animal, and leukocyte-rich plasma was obtained through dextran sedimentation. The leukocytes were washed and fixed as above.

*Immunofluorescence flow cytometry.* PMN were incubated with primary mAb against integrin molecules (10 µg/ml) for 20 min at 4°C. After three washes the PMN were incubated with FITC-conjugated goat anti-mouse (detecting P1E6, L25.3 and mAb16), goat anti-rat (detecting mAb13), or goat anti-hamster (detecting Hal/29) F(ab')₂ (Jackson Immunoresearch Lab., West Grove, PA), diluted 1:100, for 20 min at 4°C in the dark. The cells were again washed three times and analyzed on a FACSort flow cytometer (Becton Dickinson). Gating was based on forward and side scatter parameters and purity of analyzed human PMN was assured with neutrophil specific marker for CD 16 (mAb DJ130c; Dako, Glostrup, Denmark). Lymphocyte and platelet contamination was excluded by negative staining with markers for CD2 (mAb MT910; Dako) and CD41 (mAb 5B12; Dako), respectively. Purity of rat PMN, collected from the peritoneal cavity was determined through differential leukocyte count (Wright/Giemsa stain) (Werr et al., *supra*)*.* Fluorescence intensity of 1×10⁴ PMN was analyzed and compared to non-specific background fluorescence of irrelevant mouse, rat or hamster IgG.

*Identification of α₂β₁ integrin expression on human PMN adhering to collagen.* Purified native collagen (type I) from rat tail tendons, extracted according to standard procedures, was a generous gift from Dr. Björn Öbrink (CMB, Karolinska Institutet). Isolated blood PMN at a concentration of 0.5 × 10⁶ cells/ml were plated on coverslips coated with collagen, 20 µg/ml. PMN adhesion and spreading was induced by stimulation with the chemotactic peptide f-met-leu-phe (fMLP, 10⁻⁷ M; Sigma) for 15 min at 37° C. Non-adherent cells were rinsed off from the coverslips with ice-cold HBSS and adherent cells were fixed for 5 min in 4 % paraformaldehyde (Sigma) at room temperature. Immunofluorescent staining of α₂β₁ on adherent cells was performed on ice by incubation with primary mAb P1E6 (10 µg/ml) for 30 min. The samples were rinsed three times with ice-cold HBSS and incubated with FITC-conjucated anti-mouse F(ab')₂ (Jackson Immunoresearch Lab.), diluted 1:100, for additional 30 min at 4° C in the dark. The samples were rinsed and viewed in a laser scanning confocal microscope (Insight Plus, Meridian Instruments Inc, Okemos, MI) under normal transmitted and laser emitted fluorescent light. Correction for unspecific antibody binding and background fluorescence was made by comparing specific mAb fluorescence with that of samples treated with irrelevant antibodies at the same concentration and incubation time.

*Intravital time lapse videomicroscopy of PMN locomotion in vivo.* PMN locomotion in rat mesenteric tissue was studied through use of intravital time lapse videomicroscopy according to the protocol previously described in detail (Werr et al., *supra).* In brief, Wistar rats (200-250 g) were anesthetized with equal parts of fluanison/fentanyl (10/0.2 mg/ml; Hypnorm; Janssen-Cilag Ltd., Saunderton, UK) and midazolam (5 mg/ml; Dormicum; Hoffman-La Roche, Basel, Switzerland) diluted 1:1 with sterile water (2 ml/kg i.m.). Body temperature was maintained at 37° C by a heating pad connected to a rectal thermistor. After laparotomy, a segment of the ileum was exposed on a heated transparent pedestal to allow microscopic observation of the mesenteric microvasculature (Orthoplan microscope equipped with water immersion lens SW×25, NA 0.60; Leitz, Wetzlar, Germany). The microscopic image was televised and recorded on time lapse video (at 1/7 of normal speed). Analysis of leukocyte migration in the mesenteric tissue was made off line and the migration path of individual leukocytes was tracked with a digital image analyzer.

Leukocyte extravasation and migration was induced by soaking the exposed mesentery with 5 ml bicarbonate buffer solution (37° C) containing PAF at a concentration of 10⁻⁷ M. The tissue was then covered with a transparent plastic film to provide continuos chemotactic stimulation by PAF. After 40 min of chemotactic stimulation, when numerous leukocytes had extravasated, time lapse recording of leukocyte migration was undertaken, first for 20 min to assess basal migration rates in response to PAF stimulation, and then for additional 40 min in the presence of PAF together with antibodies or peptides. The antibody concentration in the mixture administered to the tissue was for all antibodies 100 µg/ml. The peptide DGEA was similarly administered at a concentration of 25 mM and SLIDIP/RGDGW at a concentration of 500 µM. Due to dilution of the reagent in fluid covering the tissue these seemingly high doses, ~10 times the documented effective blocking dose, were chosen in order to reach a sufficient concentration in proximity of the migrating leukocytes in the tissue. Cells that did not move during the observation time were not included in the analysis. As previously documented, more than 85 % of the migrating cells were neutrophils (Werr et al., *supra).*

*PMN migration in gels of collagen (type I) and gelatin.* Gels were formed in 24-well culture dishes (250 µl/well) by mixing 8.5 volumes of rat collagen solution or bovine gelatin solution (Sigma) at a concentration of 1.5 mg/ml with 1 volume of × 10 MEM (Life Technologies, Gaithersburg, MD) and 0.5 volume of 4.4 % NaHCO₃ and fMLP (final concentration 10⁻⁷ M). Purified human PMN (0.5 × 10⁶), suspended in 200 µl of MEM containing 10⁻⁹ M fMLP were placed on top of the gels and incubated with or without mAb:s or peptides for 30 min at 37° C . The final concentration was for all mAb:s 20 µg/ml, for DGEA 5 mM and for SLIDIP/RGDGW 100 µM. Four to six experiments were run in duplicate gels for each reagent tested and ten randomly chosen microscopic fields (with a defined area of 0.0625 mm²) were analyzed in each gel. The migration of PMN into the gel was analyzed with an Leitz Orthoplan microscope equipped with a water immersion lens (Leitz UO×55W, NA 0.80) by focusing down through the gel. The calibrated micrometer scale in the fine focus adjustment was used to determine the migration distance of individual PMN from the upper gel surface. The average migration distance of the three leading cells in each field was calculated and defined as the migration distance of the leading front.

### PMN accumulation in the mouse air pouch; antibody.

Male C57BL/6 mice weighing 25-30 g were anaesthetized through inhalation of Isofluran (Abbott Laboratories, North Chicago, IL), and 5 ml of sterile air was injected subcutaneously into the back. After three days, the air pouch was reinflated with 2.5 ml sterile air. Six days after the initial air injection, 0.5 ml HBSS containing PAF (10⁻⁷ M) together with mAb Ha1/29 or isotype matched control mAb (irrelevant hamster IgG) at a final concentration of 50 µg/ml was injected into the air pouch. HBSS without PAF was used to assess PMN accumulation in the pouch in absence of a chemotactic stimulus. Four hours later, the animals were killed through inhalation of Isofluran and the air pouches lavaged with 1 ml of HBSS. Leukocytes in exudates were stained and counted in a Bürker chamber.

### PMN accumulation in the mouse air pouch; α₂β₁ integrin-binding peptide.

Male C57BL/6 mice weighing 25-30 g were anaesthetized through inhalation of Isofluran (Abbott Laboratories, North Chicago, IL), and 5 ml of sterile air was injected subcutaneously into the back. After three days, the air pouch was reinflated with 2.5 ml sterile air. Six days after the initial air injection, 0.5 ml HBSS containing PAF (10⁻⁷ M) or fMLP (10⁻⁷ M) with or without peptide RKK at final concentration of 1 mM (kind gift of Dr. Johanna Ivaska, University of Turku, Finland; Ivaska et al. J. Biol. Chem 274:3513-3521, 1999) was injected into the air pouch. 3.5h later, the animals were killed through inhalation of Isofluran and the air pouches lavaged with 1 ml of HBSS. Leukocytes in exudates were stained and counted in a Bürker chamber. The results of this experiment are shown in Fig 6.

*Statistical evaluation.* Data are presented as means ± SD. Statistical significance was calculated using the Wilcoxon signed rank test for paired observations and the Mann-Whitney test for independent samples.

### Results:

*Surface expression of α₂, α₄, and α₅ integrin subunits is induced in extravasated PMN.* Surface expression of β₁ integrin receptors was analyzed on blood PMN and on extravasated human PMN obtained with the skin blister chamber technique. Immunofluorescence flow cytometry showed that expression of the β₁ integrin subunit on blood PMN is limited, and apparently associated with the α₆ subunit (data not shown). A significant increase in β₁ integrin expression was detected on extravasated PMN. Expression of the α₂, α₄ and α₅ subunits was concomitantly induced in the extravasated PMN (Fig. 1 a), whereas the expression of α₁ and α₃ remained negative (data not shown). Stimulation of isolated human blood PMN with the chemoattractant fMLP (10⁻⁹-10⁻⁵ M) or PAF (10⁻⁷-10⁻⁵ M) failed to induce α₂β₁ integrin expression (data not shown).

In agreement with the induction of α₂β₁ integrin expression on extravasated human PMN, α₂β₁ was also detected on rat PMN that accumulated in the peritoneal cavity in response to chemotactic stimulation with PAF (Fig. 1 b).

*α₂β₁ integrin is detectable on adherent PMN.* Laser-scanning confocal microscopy was used to investigate α₂β₁ integrin expression and distribution on isolated blood PMN adhering to collagen in response to stimulation with fMLP (Fig. 2). In most cells with a polarized morphology, intense staining for α₂β₁ was localized to the front of the cell (Fig. 2 b). Occasionally, weak staining was found at the rear end. For adherent PMN with a less-polarized morphology, staining for α₂β₁ was fainter and distributed diffusedly over the cell membrane.

*Blockade of α₂β₁ function inhibits PMN locomotion in rat mesenteric tissue in vivo and in collagen gels in vitro.* Direct intravital time lapse videomicroscopy was used to investigate PMN locomotion in the rat mesentery *in vivo.* The migration velocity of randomly chosen PMN that had extravasated to the interstitial tissue in response to PAF stimulation was determined before and after topical administration of function blocking antibodies or peptides against β₁ integrins (Fig. 3). Treatment with mAb Hal/29 against the rat α₂ integrin subunit rapidly and persistently reduced PMN migration velocity from 14.7 ± 1.4 µm/min in response to PAF alone to 3.9 ± 0.9 µm/min after administration of the antibody (73 ± 3 % inhibition, p<0.001) (Fig. 3a). Similar inhibition of PMN locomotion has previously been reported by us after antibody blockade of the common β₁ integrin chain (Werr et al., *supra*)*.* As shown in figure 3 a, combined administration of antibodies against the α₂ and the β₁ integrin chain did not further inhibit PMN locomotion above what was found for either of the treatments alone (16.9 ± 1.4 µm/min to 5.9 ± 0.7 µm/min, 65 ± 6 % inhibition, p<0.001). Treatment with antibodies against the α₄ or α₅ subunits was without effect on migration velocity. Also, administration of isotype matched control antibodies at the same concentration, and with the same incubation time, did not modulate PMN migration (data not shown).

β₁ integrin-binding peptides had similar activity profile on PMN migration velocity as antibodies (Fig 3b). The tetrapeptide DGEA, specifically blocking α₂β₁ integrin-mediated adhesion to collagen (Staatz et al., *supra*), inhibited PMN migration velocity by 70 ± 10 % (p<0.001) whereas peptides against α₄β₁ integrin (SLIDIP) and α₅β₁ integrin (RGDGW) were without effect on PMN migration. The use of ten-fold higher concentrations of SLIDIP/RGDGW in some experiments did not yield different effects.

We further analyzed the involvement of β₁ integrins in human PMN locomotion in collagen (type I) gels (Fig. 4a). Migration distance of the leading front offMLP- stimulated PMN was 76.5 ± 5.3 µm over a 30 min incubation period at 37°C. Blocking α₂β₁ integrin function with either mAb P1E6 or AK7, or peptide DGEA reduced the migration distance of fMLP-stimulated PMN to the level of non-stimulated cells. Antibody blockade of the common β₁ chain either alone or in combination with anti-α₂ antibodies was less effective in inhibiting PMN locomotion than blockade of α₂ alone. Blocking α₄β₁ and α₅β₁, alone or in combination, resulted in a slightly increased migration distance in the collagen gels. Qualitatively similar results were obtained with PAF-stimulated PMN (data not shown). However, migration was less efficient compared with fMLP as chemotactic stimulus, which may reflect a high capability of fMLP in comparison to PAF to rapidly establish a concentration gradient in the gels.

In a separate set of experiments, PMN migration in gelatin gels was assessed (Fig. 4b). This was important in order to verify that the modulatory effect of β₁ integrin blockade was substrate dependent. In comparison with collagen gels, fMLP-stimulated migration in gelatin was less efficient (37.6 ± 3.9µm). Notably, neither blockage of the α₂ subunit or the common β₁ chain significantly modulated the migration distance of the leading front.

*Blocking α₂β₁ integrin function reduces PAF-stimulated PMN accumulation in mouse air pouch.* The involvement of α₂β₁ in regulating chemoattractant-induced PMN accumulation in extravascular tissue was further investigated using the mouse air pouch model of acute inflammation. As shown in figure 5, significant leukocyte accumulation (>90 % PMN as identified by nuclear staining) was induced by incubating the air pouch with PAF (together with control antibody) for 4 h. Adding PAF together with the anti-α₂ mAb Ha1/29 reduced PMN accumulation by 48 ± 15 % (p<0.001). Compensating for the spontaneous PMN accumulation in the pouch (HBSS only), this value would indicate an inhibition of chemoattractant-induced PMN accumulation by approximately 70 %.

### Discussion

One hallmark of acute inflammation is the early and rapid recruitment of PMN to sites of infection and tissue injury. The initial events in the PMN extravasation process have been carefully investigated, revealing a complex receptor cross-talk between leukocytes and endothelium (Springer et al., *supra).* Less is known, however, about adhesive interactions involved in the subsequent PMN locomotion in extravascular tissue. The present inventors recently showed that adhesion molecules belonging to the β₁ integrin family participate in this process (Werr et al., *supra*). In the present application, evidence is provided for a critical role of the collagen binding α₂β₁ integrin in the recruitment of PMN to inflamed tissue sites. First, *in vivo* extravasation of human and rodent PMN is associated with induction of α₂β₁ integrin on the PMN surface.
Second, blockade of α₂β₁ integrin function with either mAb or peptide markedly impairs PMN locomotion both *in vivo* and *in vitro.* Third, PMN recruitment to tissues in response to chemotactic stimulation is greatly reduced after local anti-α₂ treatment. Surface expression of α₂β₁ was detected on both human PMN that had extravasated in the skin of the forearm and rat PMN after extravasation in the peritoneal cavity. Since expression of α₂β₁ on blood PMN is negative, these findings indicate that α₂β₁ is induced in these cells in conjunction with their emigration from blood to tissue. A similar induction of α₄β₁ expression upon PMN extravasation has previously been reported (Reinhardt et al., *supra;* Werr et al., *supra*)*.* However, the present invention is the first ever to demonstrate that α₂β₁ can be found on PMN and serves a specific function in these cells. Similar to the αᵥβ₃ integrin, which is thought to support PMN locomotion on vitronectin (Lawson MA, Maxfield FR: Ca(2+)- and calcineurin-dependent recycling of an integrin to the front of migrating neutrophils. Nature 377:75, 1995), α₂β₁ was polarized to the front of PMN migrating on collagen or in the connective tissue *in vivo.* The precise mechanisms underlying the induction of different β₁ integrins on the PMN surface in association with their extravasation are still unknown and need further investigation. Previous data have suggested that transmigration of the PMN and cytoskeletal reorganization is required for upregulation of β₁ integrins (Kubes et al., *supra*). The observation provided in the present application that chemotactic stimulation of isolated blood PMN in suspension failed to induce α₂β₁ integrin expression, whereas expression is induced in stimulated PMN adhering to collagen, further suggests that adhesion-dependent signaling events are significant for induction of β₁ integrin expression. The apparent complexity of regulation of α₂β₁ expression in PMN may explain why this cell population formerly has been considered to lack this receptor (Hemler et al., *supra).*

Chemoattractants are known to induce a kinetic response of leukocytes (chemokinesis) which in the presence of a concentration gradient is manifested as a unidirectional movement (chemotaxis). In the present application, blockage of α₂β₁ integrin function is shown to inhibit both directional (collagen gel and mouse air pouch) and random (rat mesentery) PMN locomotion which conforms with the notion that the biochemical mechanisms by which chemoattractants stimulate chemotaxis and chemokinesis probably are the same (Wilkinson PC: How do leukocytes perceive chemical gradients? FEMS Microbiol. Immunol. 2:303, 1990). Further, the present findings that inhibition of α₂β₁ integrin function suppressed PMN locomotion in both the rat mesentery *in vivo* and collagen gels *in vitro* indicate that the antibody/peptide effect on PMN migration *in vivo* was not due to potential involvement in this process of other β₁ integrin expressing cells present in the tissue (e.g. fibroblasts and mast cells). The high degree of inhibition of PMN locomotion evoked by blockade of the α₂β₁ integrin with either antibody or peptide, and the present observation that combined blockade of α₂ and the common β₁ chain did not result in further inhibition of locomotion compared with blockade of either of them alone, indicate that β₁ integrin-mediated PMN locomotion in extravascular connective tissue (Werr et al, *supra)* is primarily dependent on the α₂β₁ integrin and clearly suggest a principal role of α₂β₁ in leukocyte motility. Substrate specificity in this interaction is indicated by the differences observed for migration in collagen vs. gelatin gels. Accordingly, as previously shown by the present inventors, neither antibodies nor integrin binding peptides, blocking the function of the fibronectin binding receptors α₄β₁ and α₅β₁, are effective in modulating PMN locomotion in the rat mesentery *in vivo* (Werr et al., *supra).* Interestingly, herein is reported a tendency for slightly increased PMN migration distance in collagen gels after blockade of α₄ and α₅ integrins. Fibronectin can be secreted by activated PMN (Salcedo R, Wasserman K, Patarroyo M: Endogenous fibronectin of blood polymorphonuclear leukocytes: stimulus-induced secretion and proteolysis by cell surface-bound elastase. Exp. Cell Res. 233:33, 1997), which may explain a function of the fibronectin binding receptors in gels of pure collagen. In accordance, PMN locomotion in collagen gels has been shown to be reduced upon supplementation of the gel with fibronectin (Kuntz RM, Saltzman WM: Neutrophil motility in extracellular matrix gels: mesh size and adhesion affect speed of migration. Biophys. J. 72:1472, 1997). These data may suggest an anchoring function of the α₄ and α₅ integrins in the leukocyte extravascular migration and indicate a complex interplay between different integrin receptors in regulating the motility of these cells. The distribution of integrin receptors on the cell surface (e.g. receptor clustering and polarization) and their recycling properties, critical for receptor function and ECM-interactions, have been shown to be differentially regulated for different integrins (Bretscher MS: Circulating integrins: alpha 5 beta 1, alpha 6 beta 4 and Mac-1, but not alpha 3 beta 1, alpha 4 beta 1 or LFA-1. EMBO J. 11:405, 1992), which may explain distinct roles of various β₁ integrins in the locomotive process. Moreover, the receptor ligand avidity may be regulated distinct from the receptor expression (Huttenlocher A, Ginsberg MH, Horwitz AF: Modulation of cell migration by integrin-mediated cytoskeletal linkages and ligand-binding affinity. J. Cell Biol. 134:1551, 1996), and rate of changes in avidity may accordingly determine the role of different integrins in the consecutive steps of the leukocyte extravasation process (Weber C, Katayama J, Springer TA: Differential regulation of beta 1 and beta 2 integrin avidity by chemoattractants in eosinophils. Proc. Natl. Acad. Sci. USA 93:10939, 1996).

Others have observed increased β₁ integrin expression in T-cells after transendothelial migration (Masuyama J-I, Berman JS, Cruikshank WW, Morimoto C, Center DM: Evidence for recent as well as long term activation of T cells migrating through endothelial cell monolayers in vitro. J. Immunol. 148:1367, 1992), and that lymphocyte locomotion in collagen gels is attenuated by α₂β₁ blockade (Friedl P, Noble PB, Zänker KS: T lymphocyte locomotion in a three-dimensional collagen matrix. Expression and function of cell adhesion molecules. J. Immunol. 154:4973, 1995). These results are consistent with the present findings, and indicate that induction and engagement of α₂β₁ may be a general mechanism by which the different leukocyte subclasses reach their targets in extravascular tissue. On the other hand, integrin-independent PMN locomotion has been demonstrated in experimental systems devoid of extracellular matrix proteins (Malawista SE, de Boisfleury Chevance A: Random locomotion and chemotaxis of human polymorphonuclear leukocytes (PMN) in the presence of EDTA: PMN in close quarters require neither leukocyte integrins nor external divalent cations. Proc. Natl. Acad. Sci. USA 94:11577, 1997). These observations indicate that integrin receptors, apparently required for PMN locomotion in the dense restraining meshwork of biopolymers in native tissue, may be of little importance for locomotion in an environment lacking such elements. Noteworthy, collagen, by far, is the most abundant protein in the extracellular matrix and makes up one third of total body protein which speaks in favor of collagen as a primary substrate in leukocyte interactions with extracellular matrix.

Using the mouse air pouch model of acute inflammation, it was shown that PMN recruitment to tissues in response to chemotactic stimulation is markedly suppressed after local treatment with monoclonal antibodies against the α₂β₁ integrin. Since mAb treatment was extravascular, and uptake of intact mAb into the circulation is limited due to the size of the Ig molecule, the inhibitory effect on PMN accumulation in the pouch most likely can be ascribed to an extravascular activity of the mAb in agreement with our microscopic observations rather than being related to an effect on intravascular events. Together with the microscopic findings of impaired PMN locomotion after α₂ blockade, these observations indicate a direct relationship between the locomotive capacity of PMN in the extravascular space and their ability to accumulate in inflamed tissue. Thus, the α₂β₁ integrin complements the intravascular functions of the selectins and β₂ integrins in the recruitment of PMN to sites of injury or infection by serving a distinct and critical function in the extravascular phase of this process. These findings provide new insight into the roles of various cell adhesion molecules in leukocyte trafficking, and they also suggest that the α₂β₁ integrin is a potential target molecule in the development of new therapeutic strategies in treatment of inflammatory disease.

## Claims

1. Use of an α₂β₁-integrin suppressor or inhibitor, which is an anti-α₂β₁-integrin antibody or an α₂β₁-integrin binding peptide, for the manufacture of a medicament for the prevention and/or treatment of an inflammatory condition selected from the group consisting of inflammatory bowel disease (IBD), asthma and psoriasis.

2. Use according to claim 1, wherein the anti-α₂β₁-integrin antibody is a monoclonal anti-α₂β₁-integrin antibody.

3. Use according to claim 1-2, wherein the antibody is targeted against the α₂-subunit of the α₂β₁-integrin.

4. Use according to claim 1, wherein the α₂β₁-integrin binding peptide comprises the RKK sequence.

5. Use according to claim 1, wherein the α₂β₁-integrin binding peptide comprises the DGEA sequence.

6. Use according to any of the preceding claims for the manufacture of a medicament in a form suitable for local administration.

7. Use according to any of the preceding claims for the manufacture of a medicament in a form suitable for rectal administration.

8. Use according to any of the preceding claims, wherein the IBD is ulcerative colitis and/or Crohn's disease.

9. A pharmaceutical preparation comprising an α₂β₁-integrin binding peptide which suppresses or inhibits α₂β₁-integrin function and a pharmaceutically acceptable carrier thereof.

10. The pharmaceutical composition according to claim 9, wherein the α₂β₁-integrin binding peptide comprises the RKK sequence.

11. The pharmaceutical composition according to claim 9, wherein the α₂β₁-integrin binding peptide comprises the DGEA sequence.

12. An *in vitro* method for assessment of the progression and stage of inflammatory bowel disease (IBD), wherein
a. an α₂β₁-integrin suppressor or inhibitor, which is an anti-α₂β₁-integrin antibody or an α₂β₁-integrin binding peptide, is contacted with a sample; and
b. the amount of α₂β₁-integrin bound by said anti-α₂β₁-integrin antibody or an α₂β₁-integrin binding peptide is measured, said amount being an indication on the progression and stage of said condition.

13. The method according to claim 12, wherein said anti-α₂β₁-integrin antibody is a monoclonal anti-α₂β₁-integrin antibody.

14. The method according to claim 12-13, wherein said anti-α₂β₁-integrin antibody is targeted against the α₂-subunit of the α₂β₁-integrin.

15. The method according to claim 12, wherein the α₂β₁-integrin binding peptide includes the RKK sequence.

16. The method according to claim 12, wherein the α₂β₁-integrin binding peptide includes the DGEA sequence.

17. The method according to the claims 12-16, wherein the IBD is ulcerative colitis and/or Crohn's disease.

## Patentansprüche

1. Verwendung eines α₂β₁-Integrinsuppressors oder -hemmers, der ein anti-α₂β₁-Integrinantikörper oder ein α₂β₁-Integrin bindendes Peptid ist, zur Herstellung eines Arzneimittels zur Prävention und/oder Behandlung eines entzündlichen Zustandes, der aus der Gruppe ausgewählt wird, die aus chronisch-entzündlichen Darmerkrankungen (C-E-D), Asthma und Psoriasis besteht.

2. Verwendung nach Anspruch 1, wobei der anti-α₂β₁-Integrinantikörper ein monoklonaler anti-α₂β₁-Integrinantikörper ist.

3. Verwendung nach Ansprüchen 1-2, wobei der Antikörper gegen die α₂-Untereinheit des α₂β₁-Integrins gerichtet ist.

4. Verwendung nach Anspruch 1, wobei das α₂β₁-Integrin bindende Peptid die RKK Sequenz umfasst.

5. Verwendung nach Anspruch 1, wobei das α₂β₁-Integrin bindende Peptid die DGEA Sequenz umfasst.

6. Verwendung nach einem der vorangehenden Ansprüche zur Herstellung eines Arzneimittels in einer Form, die für lokale Verabreichung geeignet ist.

7. Verwendung nach einem der vorangehenden Ansprüche zur Herstellung eines Arzneimittels in einer Form, die für rektale Verabreichung geeignet ist.

8. Verwendung nach einem der vorangehenden Ansprüche, wobei die C-E-D Colitis ulcerosa und/oder Morbus Crohn ist.

9. Pharmazeutische Zubereitung, die ein α₂β₁-Integrin bindendes Peptid, welches die Funktion des α₂β₁-Integrin unterdrückt oder hemmt, und einen pharmazeutisch annehmbaren Träger dafür umfasst.

10. Pharmazeutische Zusammensetzung nach Anspruch 9, wobei das α₂β₁-Integrin bindende Peptid die RKK Sequenz umfasst.

11. Pharmazeutische Zusammensetzung nach Anspruch 9, wobei das α₂β₁-Integrin bindende Peptid die DGEA Sequenz umfasst.

12. *In vitro* Verfahren zur Bestimmung des Fortschreitens und des Stadiums von chronisch-entzündlicher Darmerkrankung (C-E-D), wobei
a. ein α₂β₁-Integrinsuppressor oder -hemmer, der ein anti-α₂β₁-Integrinantikörper oder ein α₂β₁-Integrin bindendes Peptid ist, mit einer Probe in Kontakt gebracht wird; und
b. die Menge an α₂β₁-Integrin gemessen wird, die durch den anti-α₂β₁-Integrinantikörper oder ein α₂β₁-Integrin bindende Peptid gebunden wird, wobei diese Menge eine Indikation für das Fortschreiten und das Stadium der Erkrankung ist.

13. Verfahren nach Anspruch 12, wobei der anti-α₂β₁-Integrinantikörper ein monoklonaler anti-α₂β₁-Integrinantikörper ist.

14. Verfahren nach Ansprüchen 12-13, wobei der anti-α₂β₁-Integrinantikörper gegen die α₂-Untereinheit des α₂β₁-Integrins gerichtet ist.

15. Verfahren nach Anspruch 12, wobei das α₂β₁-Integrin bindende Peptid die RKK Sequenz umfasst.

16. Verfahren nach Anspruch 12, wobei das α₂β₁-Integrin bindende Peptid die DGEA Sequenz umfasst.

17. Verfahren nach Ansprüchen 12-16, wobei die C-E-D Colitis ulcerosa und/oder Morbus Crohn ist.

## Revendications

1. Utilisation d'un suppresseur ou d'un inhibiteur de l'intégrine α₂β₁, qui est un anticorps anti-intégrine α₂β₁ ou un peptide de liaison avec l' intégrine α₂β₁, pour la production d'un médicament destiné à la prévention et/ou au traitement d'une affection inflammatoire sélectionnée parmi le groupe constitué par la maladie intestinale inflammatoire (MII), l'asthme et le psoriasis.

2. Utilisation selon la revendication 1, dans laquelle l'anticorps anti-intégrine α₂β₁ est un anticorps anti-intégrine α₂β₁ monoclonal.

3. Utilisation selon les revendications 1 et 2, dans laquelle l'anticorps est ciblé sur la sous-unité α₂ de l'intégrine α₂β₁.

4. Utilisation selon la revendication 1, dans laquelle le peptide de liaison avec l'intégrine α₂β₁ comprend la séquence RKK.

5. Utilisation selon la revendication 1, dans laquelle le peptide de liaison avec l'intégrine α₂β₁ comprend la séquence DGEA.

6. Utilisation selon l'une quelconque des revendications précédentes pour la fabrication d'un médicament sous une forme appropriée pour l'administration locale.

7. Utilisation selon l'une quelconque des revendications précédentes pour la fabrication d'un médicament sous une forme appropriée pour l'administration rectale.

8. Utilisation selon l'une quelconque des revendications précédentes, dans laquelle la MII est la colite ulcéreuse et/ou la maladie de Crohn.

9. Composition pharmaceutique comprenant un peptide de liaison avec l'intégrine α₂β₁ qui supprime ou inhibe la fonction de l'intégrine α₂β₁ et un vecteur pharmaceutiquement acceptable de celui-ci.

10. Composition pharmaceutique selon la revendication 9, dans laquelle le peptide de liaison avec l'intégrine α₂β₁ comprend la séquence RKK.

11. Composition pharmaceutique selon la revendication 9, dans laquelle le peptide de liaison avec l'intégrine α₂β₁ comprend la séquence DGEA.

12. Procédé *in vitro* pour l'évaluation de la progression et du stade de la maladie intestinale inflammatoire (MII), dans laquelle
a. un suppresseur ou un inhibiteur de l'intégrine α₂β₁, qui est un anticorps anti-intégrine α₂β₁ ou un peptide de liaison avec l'intégrine α₂β₁, est mis en contact avec un échantillon ; et
b. la quantité d'intégrine α₂β₁ liée par ledit anticorps anti-intégrine α₂β₁ ou un peptide de liaison avec l'intégrine α₂β₁ est mesurée, ladite quantité étant une indication de la progression et du stade de ladite affection.

13. Procédé selon la revendication 12, dans lequel ledit anticorps anti-intégrine α₂β₁ est un anticorps anti-intégrine α₂β₁ monoclonal.

14. Procédé selon les revendications 12 et 13, dans lequel ledit anticorps anti-intégrine α₂β₁ est ciblé sur la sous-unité α₂ de l'intégrine α₂β₁.

15. Procédé selon la revendication 12, dans lequel le peptide de liaison avec l'intégrine α₂β₁ comprend la séquence RKK.

16. Procédé selon la revendication 12, dans lequel le peptide de liaison avec l'intégrine α₂β₁ comprend la séquence DGEA.

17. Procédé selon les revendications 12 à 16, dans lequel la MII est la colite ulcéreuse et/ou la maladie de Crohn.
